# EUROPÄISCHE PATENTANMELDUNG

(11) **EP 0 998 879 A1**
(43) Veröffentlichungstag der Anmeldung: **10.05.2000**
(21) Anmeldenummer: 99120348.0
(22) Anmeldetag: 12.10.1999
(51) Int. Cl.: A61B 18/14

(54) **Instrument für die Hochfrequenzchirurgie**

(30) Priorität: 03.11.1998 DE 19850663
(71) Anmelder: Erbe Elektromedizin GmbH., D-72072 Tübingen (DE)
(72) Erfinder: Fischer, Klaus, 72202 Nagold (DE); Bergler, Wolfgang, 68161 Mannheim (DE); Farin, Günter, 72070 Tübingen (DE)
(74) Vertreter: Bohnenberger, Johannes, Dr.

(57) **Zusammenfassung**

Es wird ein Instrument für die Hochfrequenzchirurgie aufgezeigt, das einen Handhabungsgriff und ein Präparationsinstrument mit spatel- oder löffelförmigen Flächenbereichen an einem distalen Abschnitt des Handhabungsgriffs zur mechanischen und/oder hochfrequenzchirurgischen Präparierung von biologischem Gewebe aufweist. Die Flächenbereiche umfassen eine innere und eine äußere Fläche, von denen mindestens die innere Fläche elektrisch isoliert ist. Es ist mindestens eine elektrisch leitfähige als Elektrode ausgebildete und mit einem HF-Generator verbundene Randzone zwischen der inneren und der äußeren Fläche vorgesehen.

## Beschreibung

Die Erfindung betrifft ein Instrument für die Hochfrequenzchirurgie, das zur mechanischen und - alternativ - hochfrequenzchirurgischen Präparierung biologischer Gewebestrukturen sowie zum Blutstillen geeignet ist.

Aus der DE 34 12 756 ist ein Arthroskopiemesser bekannt, dessen hakenförmige "Klinge" bis auf einen schmalen Bereich mit einer isolierenden Dentalkeramik überzogen ist, wobei der nichtisolierende Bereich bei Zuführung eines Hochfrequenzstromes eine Schneidefunktion ausübt. Dieses Gerät ist somit extrem spezialisiert und kann nur für ganz besondere Zwecke eingesetzt werden.

Der Erfindung liegt die Aufgabe zugrunde, ein Instrument aufzuzeigen, was sowohl für die Hochfrequenzchirurgie als auch für eine mechanische Präparation verwendbar ist.

Diese Aufgabe wird durch das im Patentanspruch 1 erläuterte Instrument gelöst.

Ein wesentlicher Punkt der Erfindung liegt darin, daß ein von seiner Form her an sich bekanntes Instrument eine Zusatzfunktion dadurch erhält, daß die an sich schon mechanisch wirkende Schneide nunmehr zusätzlich auch zum Schneiden mittels eines Hochfrequenzstromes oder aber zum Koagulieren von Gewebe verwendbar ist. Wichtig ist hierbei auch die Tatsache, daß eine Schneide an sich dazu gedacht ist, Gewebe zu trennen, während bei Zuführung eines Koagulationsstroms eben dieselbe Schneide auch dazu geeignet ist, Gewebe nicht zu trennen sondern eben zu koagulieren.

Vorzugsweise ist mindestens eine Elektrode somit als Schneidebereich im wesentlichen direkt an die innere, isolierende Fläche, die spatel- oder löffelförmig ausgebildet ist, angrenzend vorgesehen.

Vorzugsweise ist die mindestens eine Elektrode an einem, dem Handhabungsgriff abgewandten distalen Ende des Präparationsinstruments vorgesehen, so daß die seitlichen Randbereiche des Präparationsinstrumentes auch beim Zuführen eines Hochfrequenzstromes keine Wirkung auf das Gewebe ausüben. Die Handhabung des Gerätes wird dadurch wesentlich erleichtert.

Bei einer bevorzugten Ausführungsform ist mindestens eine zweite Elektrode an die äußere Fläche angrenzend oder diese mindestens abschnittsweise mitumfassend vorgesehen, so daß man einen bipolaren, multipolaren oder quasibipolaren Schneidvorgang durchführen kann, der keine ortsfest am Patienten angebrachte Elektrode erfordert. Überraschenderweise ist das Instrument auch in dieser Funktion verwendbar, was in erster Linie darauf zurückzuführen ist, daß man die beiden Elektroden sehr nahe beieinander anbringen kann.

Vorzugsweise ist das Präparationsinstrument aus leitfähigem Material, insbesondere aus Metall gefertigt und bis auf die Elektroden mit isolierendem Material beschichtet. Auf diese Weise ist ein Instrument mit sehr hoher Präzision herstellbar.

Das Präparationsinstrument weist vorzugsweise eine innere und eine äußere Schale aus leitfähigem Material und eine die beiden Schalen mechanisch verbindende Zwischenschicht aus elektrisch isolierendem Material auf, so daß zwei Elektroden gebildet werden können.

Bei einer weiteren bevorzugten Ausführungsform des Instrumentes ist ein Rohrleitungsabschnitt am Präparationsinstrument befestigt, dessen distale Öffnung in der Nähe eines vorderen, dem Handhabungsgriff abgewandten Ende liegt. Ein proximales Ende des Rohrleitungsabschnitts ist mit einer Einrichtung zum Zuführen eines Spülmediums, zum Absaugen von Flüssigkeit oder auch zum Zuführen eines inerten Schutzgases verbunden, so daß beim hochfrequenzchirurgischen Schneiden unter Zufuhr von Inertgas kein Rauch mehr entsteht.

Bevorzugte Ausführungsformen der Erfindung ergeben sich aus den Unteransprüchen.

Nachfolgend werden Ausführungsbeispiele der Erfindung anhand von Abbildungen näher erläutert. Hierbei zeigen:
- Fig. 1: eine Seitenansicht eines Instrumentes,
- Fig. 2: eine Draufsicht auf den vorderen Bereich des Instrumentes nach Fig. 1,
- Fig. 3: einen Schnitt entlang der Linie III/III aus Fig. 2,
- Fig. 4-6: prinzipielle Darstellungen der Endbereiche des Instrumentes zur Erläuterung der Elektrodenanordnung,
- Fig. 7: eine Seitenansicht einer weiteren Ausführungsform der Erfindung,
- Fig. 8: eine Draufsicht auf den Endabschnitt des Instrumentes nach Fig. 7,
- Fig. 9: einen Schnitt entlang der Linie VIII/VIIII aus Fig. 8,
- Fig. 10: eine Draufsicht auf eine weitere Ausführungsform eines Endabschnittes der Erfindung,
- Fig. 11: eine Seitenansicht einer weiteren Ausführungsform der Erfindung und
- Fig. 12: einen Teil-Längsschnitt des Endabschnittes des Instrumentes nach Fig. 11.

In der nachfolgenden Beschreibung werden für gleiche und gleich wirkende Teile dieselben Bezugsziffern verwendet.

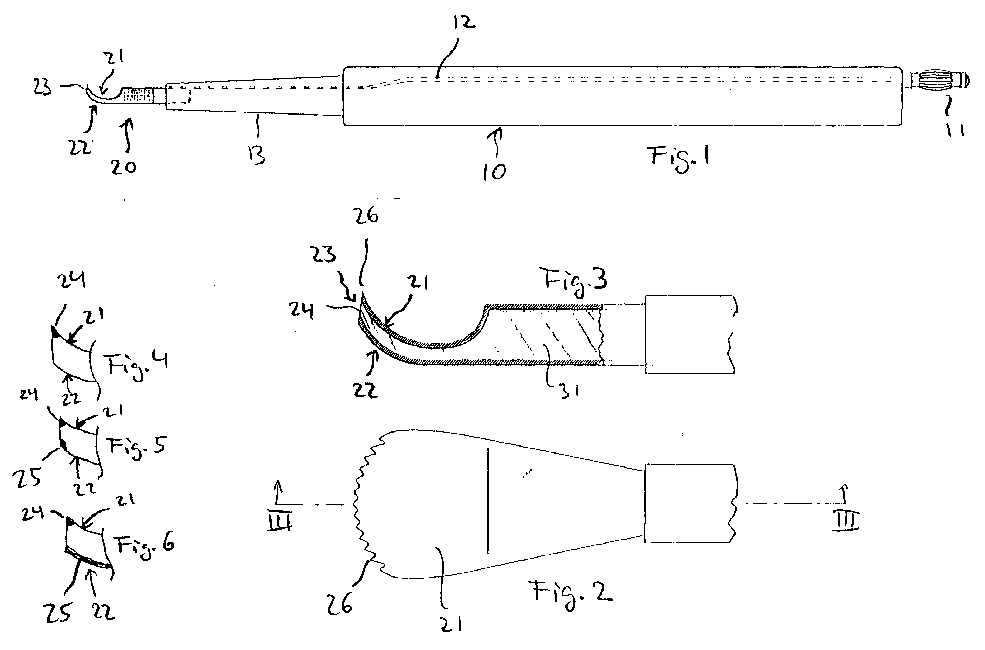

Bei der in Fig. 1 gezeigten Ausführungsform ist an einem Handhabungsgriff 10 ein Präparationsinstrument 20 über ein konisches zwischenstück 13 befestigt. Das Präparationsinstrument 20 umfaßt, wie dies aus Fig. 2 und Fig. 3 hervorgeht, eine innere Fläche 21 und eine äußere Fläche 22, die jeweils aus einem elektrisch nicht leitenden Material gefertigt oder mit einem solchen Material überzogen sind. Zwischen der inneren Fläche 21 und der äußeren Fläche 22 ist ein aus Metall bestehendes Innenteil 31 angebracht, das an seinem distalen, dem Handhabungsabschnitt 10 abgewandten Ende eine freiliegende Randzone 23 zwischen der inneren und der äußeren Fläche 21, 22 bildet. Dieses freiliegende Stück stellt eine erste Elektrode 24 dar, welche über das Innenteil 31 und eine Leitung 12 mit einer Anschlußbuchse 11 am proximalen Ende des Handhabungsgriffs 10 in Verbindung steht. Die innere Fläche 21 ist an ihrem distalen Ende, also dort, wo sie in die Randzone 23 übergeht, mit einer Schneide 26 versehen, die, wie in Fig. 2 gezeigt, gezähnt oder aber auch glatt ausgebildet sein kann.

Das so ausgebildete Instrument kann zum mechanischen Präparieren in an sich bekannter Weise verwendet werden. Wenn ein Hochfrequenzstrom zugeführt wird, so kann - je nach "Art" des Hochfrequenzstroms die Elektrode 24 zum Schneiden oder aber auch zum Koagulieren von Gewebe verwendet werden. Bei dieser Ausführungsform der Erfindung ist der Patient über eine Flächenelektrode an den zweiten Pol der Hochfrequenzquelle angeschlossen, so daß ein monopolares Instrument vorliegt.

In Fig. 4 ist schematisiert dargestellt, daß die erste Elektrode 24 auch selbst als Schneide ausgebildet sein kann.

Bei der in Fig. 5 gezeigten prinzipiellen Darstellung ist neben der ersten Elektrode 24 noch eine zweite Elektrode 25 vorgesehen, wobei die beiden Elektroden derart an die Hochfrequenzquelle angeschlossen sind, daß ein bipolares Instrument entsteht.

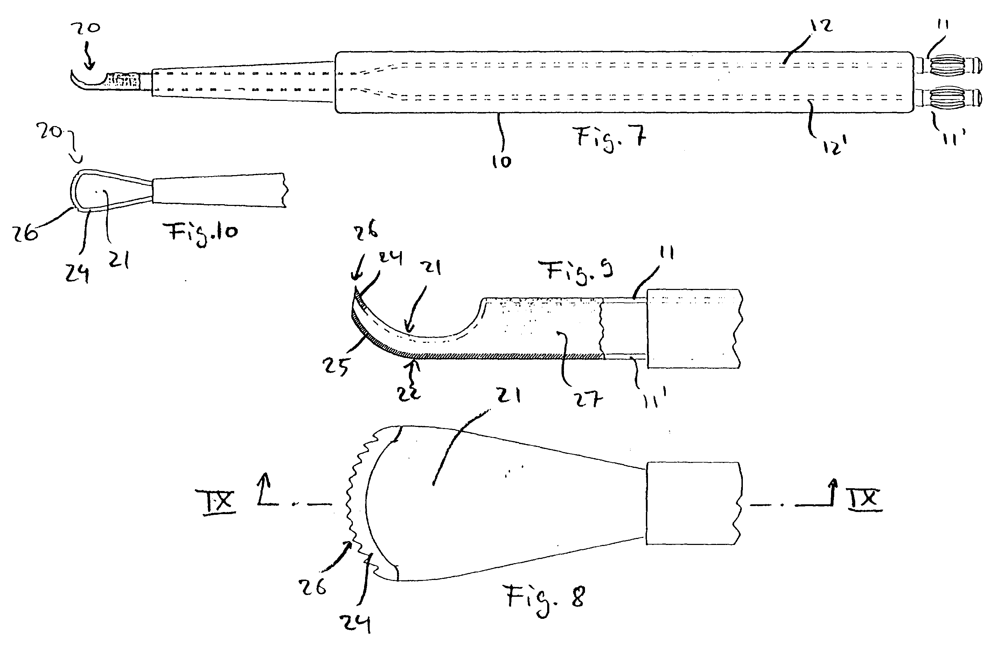

Bei der in Fig. 6 gezeigten Ausführungsform ist die zweite Elektrode 25 auf einem größeren Abschnitt oder auf der gesamten äußeren Fläche 22 ausgebildet.

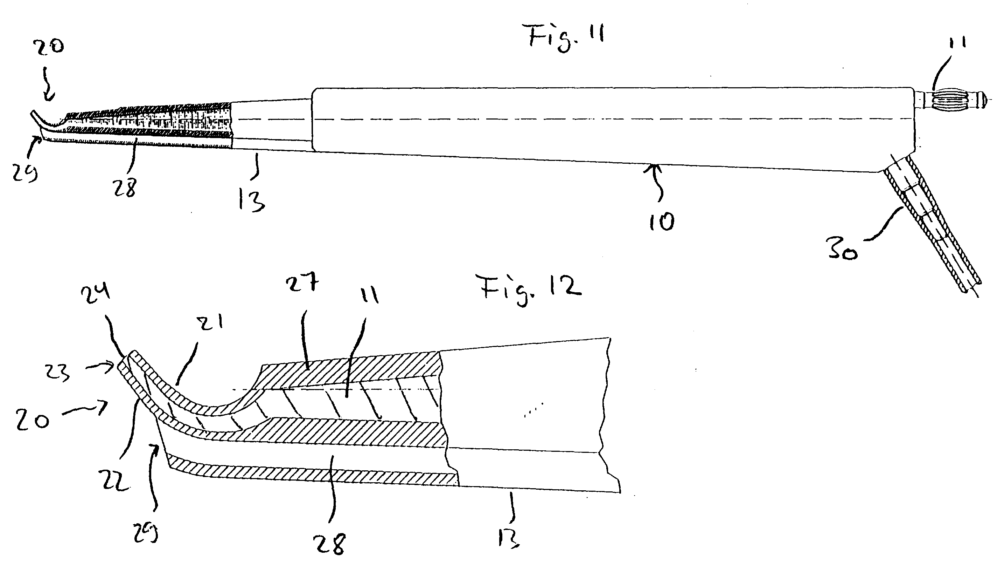

Bei der in Fig. 7 gezeigten Ausführungsform der Erfindung sind, wie in den Figuren 5 und 6 angedeutet, zwei Elektroden 24, 25 vorgesehen, so daß im Handhabungsgriff 10 auch zwei Leitungen 12, 12' mit zwei Anschlußbuchsen 11, 11' angebracht sind. Die erste Elektrode 24 ist, wie in den Figuren 8 und 9 gezeigt, die Schneide 26 bildend, im distalen Randbereich der inneren Fläche 21 ausgeformt. Elektrisch gesehen entspricht diese Ausführungsform der Erfindung derjenigen nach Fig. 6.

Bei der in Fig. 10 gezeigten Ausführungsform der Erfindung ist die erste Elektrode 24 die innere Fläche 21 umgebend ausgebildet, so daß auch mit den Seitenbereichen des Präparationsinstrumentes 20 geschnitten bzw. insbesondere koaguliert werden kann.

Bei der in den Figuren 11 und 12 gezeigten Ausführungsform ist wieder eine Elektrodenanordnung ähnlich der nach Fig. 3 vorgesehen, die Elektrode 24 liegt also zwischen der inneren Fläche 21 und der äußeren Fläche 22, die jeweils aus isolierendem Material 27 ausgebildet sind. Kurz hinter der Randzone 23 zwischen der inneren Fläche 21 und der äußeren Fläche 22 befindet sich eine distale Öffnung 29 eines Rohrleitungsabschnittes 28, der durch das konische Zwischenstück 13 und den Handhabungsgriff 10 hindurch zu einem Anschluß 30 geführt ist. Dieser Rohrleitungsabschnitt 28 kann nun in vielfältiger Weise verwendet werden. Man kann sowohl ein Spülmedium zuführen als auch Flüssigkeiten (z. B. eben dieses Spülmedium) wieder absaugen. Bei einer anderen bevorzugten Ausführungsform der Erfindung wird über den Rohrleitungsabschnitt 28 ein Inertgas zugeführt, so daß beim Hochfrequenzschneiden die Schnittstelle von Sauerstoff freigehalten wird, so daß kein Rauch entsteht. Selbstverständlich ist die Form des Präparationsinstruments bzw. die Anordnung der Elektroden, wie sie in den Figuren 11 und 12 gezeigt ist, auch anders wählbar, insbesondere so, wie es in den Figuren 1-10 gezeigt ist. Das Integrieren eines Rohrleitungsabschnitts 28 in das Präparationsinstrument ist also ein ganz allgemein gültiger Gedanke.

### Bezugszeichenliste

- 10: Handhabungsabschnitt
- 11, 11': Anschlußbuchse
- 12, 12': Leitung
- 13: konisches Zwischenstück
- 20: Präparationsinstrument
- 21: innere Fläche
- 22: äußere Fläche
- 23: Randzone
- 24: erste Elektrode
- 25: zweite Elektrode
- 26: Schneide
- 27: isolierendes Material
- 28: Rohrleitungsabschnitt
- 29: distale Öffnung
- 30: Anschlußstück
- 31: Innenteil

## Patentansprüche

1. Instrument für die Hochfrequenzchirurgie, umfassend einen Handhabungsgriff (10), ein Präparationsinstrument (20) mit spatel- oder löffelförmigen Flächenbereichen an einem distalen Abschnitt des Handhabungsgriffes (10) zur mechanischen und/oder hochfrequenzchirurgischen Präparierung von Gewebe, wobei die Flächenbereiche eine innere (21) und eine äußere Fläche (22) umfassen, von denen mindestens die innere Fläche (21) elektrisch isoliert ist und wobei mindestens eine elektrisch leitfähige, als Elektrode (24, 25) ausgebildete und mit einem HF-Generator verbundene Randzone (23) zwischen der inneren (21) und der äußeren Fläche (22) vorgesehen ist.

2. Instrument nach Anspruch 1,
**dadurch gekennzeichnet,**
daß die mindestens eine Elektrode (24) als Schneidebereich (26) im wesentlichen direkt an die innere, isolierende Fläche (21) angrenzend vorgesehen ist.

3. Instrument nach einem der vorherigen Ansprüche,
**dadurch gekennzeichnet,**
daß die mindestens eine Elektrode (24) an einem, dem Handhabungsgriff (10) abgewandten distalen Ende des Präparationsinstruments (10) angebracht ist.

4. Instrument nach einem der vorherigen Ansprüche,
**dadurch gekennzeichnet,**
daß mindestens eine zweite Elektrode (25) an die äußere Fläche (22) angrenzend oder diese mindestens abschnittsweise mitumfassend zur Durchführung eines bipolaren, multipolaren oder quasibipolaren Schneidevorgangs vorgesehen ist.

5. Instrument nach einem der vorherigen Ansprüche,
**dadurch gekennzeichnet,**
daß das Präparationsinstrument (20) aus elektrisch leitfähigem Material, insbesondere aus Metall gefertigt und bis auf die Elektroden (24, 25) mit isolierendem Material (27) beschichtet ist.

6. Instrument nach einem der vorherigen Ansprüche,
**dadurch gekennzeichnet,**
daß das Präparationsinstrument (20) eine innere und eine äußere Schale aus leitfähigem Material und eine, die beiden Schalen mechanisch verbindende Zwischenschicht aus elektrisch isolierendem Material (27) umfaßt.

7. Instrument nach einem der vorherigen Ansprüche,
**gekennzeichnet durch**
einen Rohrleitungsabschnitt (28), der am Präparationsinstrument (20) befestigt ist und eine distale Öffnung (29) in der Nähe eines vorderen, dem Handhabungsgriff (10) abgewandten Ende und ein proximales Ende (30) aufweist, das mit einer Einrichtung zum Zuführen eines Spülmediums und/oder zum Absaugen von Flüssigkeit und/oder zum Zuführen eines inerten Schutzgases verbunden ist.
